# EUROPÄISCHE PATENTSCHRIFT

(11) **EP 3 008 460 B1**
(45) Veröffentlichungstag und Bekanntmachung des Hinweises auf die Patenterteilung: **03.07.2019**
(21) Anmeldenummer: 14727462.5
(22) Anmeldetag: 28.05.2014
(51) Int. Cl.: G01N 27/407

(54) **GASSENSOR ZUR MESSUNG UNTERSCHIEDLICHER GASE UND DAZUGEHÖRIGES GASMESSVERFAHREN**
GAS SENSOR FOR MEASURING DIFFERENT GASES AND ASSOCIATED METHOD FOR GAS MEASUREMENT
CAPTEUR DE GAZ POUR MESURER DIFFÉRENTS GAZ, ET PROCÉDÉ DE MESURE DE GAZ CORRESPONDANT

(30) Priorität: 11.06.2013 DE 102013210903
(43) Veröffentlichungstag der Anmeldung: 20.04.2016
(73) Patentinhaber: Heraeus Nexensos GmbH, 63450 Hanau (DE)
(72) Erfinder: WIENAND, Karlheinz, 63741 Aschaffenburg (DE); ZINKEVICH, Matsvei, 63773 Goldbach (DE); ULLRICH, Karl-Heinz, 63920 Großheubach (DE)
(74) Vertreter: Heraeus IP
(86) Internationale Anmeldenummer: PCT/EP2014/061065
(87) Internationale Veröffentlichungsnummer: WO 2014/198540

(56) Entgegenhaltungen:
- DE-A1-102007 048 049
- DE-A1-102010 040 194
- US-A1- 2002 108 856

## Beschreibung

Die Erfindung betrifft einen Gassensor zur Messung der Konzentrationen von Gasen, insbesondere von Sauerstoff und zumindest von einem weiteren Gas, vorzugsweise zumindest von einer oxidierbaren Abgaskomponente in Form eines weiteren Gases, aufweisend zumindest einen Festkörperelektrolyten aus zumindest 90 Gewichtsprozent ZrO₂ und Rest zumindest aus einem weiteren Element aus der Gruppe bestehend aus Yttriumoxid und Hafniumoxid, ferner aufweisend zumindest drei Elektroden, und zwar zumindest zwei Elektroden aus dotiertem Platin und eine Elektrode aus einer Goldlegierung, wobei zwei Elektroden, die eine Elektrode aus dem dotierten Platin und die eine Elektrode aus der Goldlegierung, auf einer Oberseite des Festkörperelektrolyten und die andere Elektrode aus dem dotierten Platin auf einer der Oberseite gegenüberliegenden Unterseite des Festkörperelektrolyten angeordnet sind, und ferner aufweisend zumindest eine geschlossene Kammer, wobei die Unterseite des Festkörperelektrolyten einen Teil der Kammer bildet.

Des Weiteren betrifft die Erfindung ein Verfahren zur Messung der Konzentrationen von Gasen, eine Gassensorvorrichtung und die Verwendung eines Gassensors.

Aus der DE 196 51 328 B4 ist bekannt, dass Sauerstoffpumpen auf einer elektrochemischen Reaktion von Zirkoniumdioxid (ZrO₂) zwischen zwei Elektroden basieren. Bei der Sauerstoffpumpe wird Sauerstoff von einer Seite einer Zirkoniumdioxidwand über dessen Reduktion zu Sauerstoffionen an der Kathode, eine anschließende lonenwanderung der Sauerstoffionen im Zirkondioxid und schließlich der Oxidation der Sauerstoffionen zu Sauerstoff an der Anode zur anderen Seite transportiert bzw. gepumpt. Aufgrund dieses Effektes ist ein unterschiedlicher Partialdruck an beiden Seiten der Sauerstoffpumpe erzielbar.

Aus der DE 10 2007 048 049 A1 ist ein Gassensor bekannt, der die Messung von Abgasbestandteilen ermöglicht. Der Gassensor weist ein Substrat mit elektrisch isolierender Oberfläche, beispielsweise ein Körper aus Al₂O₃, auf. In der Schrift ist der Körper mit einem Heizwiderstand beschichtet, auf dem ein Deckel aus einem lonenleiter, gemäß der DE 10 2007 048 049 A1 ein lonenleiter aus Zirkoniumdioxid, und die Substratoberfläche einen Hohlraum erzeugen. Ober- und unterhalb des lonenleiters sind metallisch leitende Elektroden angeordnet, von denen zwei als Platinelektroden ausgeführt sind. Ferner weist der Gassensor auf dem lonenleiter eine weitere Elektrode auf, die als Mischpotentialelektrode ausgebildet ist und eine Edelmetalllegierung aufweist, die aus Gold und Platin besteht. Die Elektroden sind dabei porös, so dass sie sauerstoffdurchlässig sind. Vorzugsweise werden sie per Siebdruck aufgetragen.

Die DE 197 57 112 A1 offenbart einen Gassensor zur Messung von Sauerstoff und/oder der Luft/Kraftstoff Verhältniszahl Lambda und mindestens einem weiteren gasförmigen Bestandteil wie beispielsweise Kohlenwasserstoffe oder Stickoxide, in Gasgemischen mit einer einen konstanten Sauerstoffpartialdruck repräsentierenden Referenzelektrode, einem sauerstoffionenleitenden Festelektrolyten und mindestens zwei Messelektroden, wobei die Messelektroden und die Referenzelektrode unmittelbar auf dem Festelektrolyten angeordnet sind, und mit elektrischen Leitungen zum Anschluss und zum Abgriff für die elektrischen Messsignale. Gemäß der DE 197 57 112 A1 ist der Festelektrolyt in beliebiger geometrischer Form mit einer dem Messgas zugewandten Seite und einer vom Messgas getrennten Referenzgasseite ausgebildet, wobei die Anordnung der Elektroden mit der Referenzelektrode auf der Referenzgasseite und den mindestens zwei Messelektroden auf der Messgasseite gleichzeitig mindestens zwei, auf gleiche oder unterschiedliche Messprinzipien basierende Messsignale liefert, denen unterschiedliche gasförmige Bestandteile zugrunde liegen. Der Sensor wird beispielsweise für die Motorsteuerung von Kraftfahrzeugen verwendet.

Nachteilig bei dem heutzutage bekannten Stand der Technik ist, dass bei der Detektion der Konzentrationen von Gasen, beispielsweise die Konzentrationen von Gasen in Abgasen, eine präzise und schnelle Messung schwierig, zeitaufwendig und sehr aufwendig ist.

Aufgabe der Erfindung ist es daher, eine Möglichkeit zu schaffen, präzise, einfach und schnell die Konzentrationen von unterschiedlichen Gasen und/oder sich verwechselnden Gaszusammensetzungen zu messen.

Die Aufgabe wird dadurch gelöst, dass das dotierte Platin zwischen 0,5 Gewichtsprozent bis 15 Gewichtsprozent ZrO₂ und Rest Platin aufweist, und dass die Goldlegierung etwa 85 Gewichtsprozent Gold und etwa 15 Gewichtsprozent Platin oder die Goldlegierung Gold und Platin im Verhältnis von 85 % Gold zu 15 % Platin und zusätzlich zumindest 0,5 Gewichtsprozent bis 15 Gewichtsprozent ZrO₂ aufweist.

Die abhängigen Ansprüche enthalten bevorzugte Ausführungen gemäß der Erfindung.

Vorteilhaft ist, dass mittels der erfindungsgemäßen Anteile, nämlich des dotierten Platins und der Goldlegierung, eine schnelle, einfache und präzise Messung der Gaskonzentrationen möglich ist.

Es ist möglich, reduzierende Gase, beispielsweise Kohlenmonoxid, zum Beispiel in einem Abgas einer Heizungsanlage in dessen Abgasrohr unabhängig von der Jahreszeit und somit der Außentemperatur und der Gas-Abgaszusammensetzung zu messen. Dadurch lassen sich beispielsweise unterschiedliche Gas-Abgaszusammensetzungen, die beispielsweise bei skandinavischem Gas und russischem Gas vorliegen, durch die Messung mit Hilfe des erfindungsgemäßen Gassensors und somit auch mittels der erfindungsgemäßen Gassensorvorrichtung messen.

Die unterhalb des Festkörperelektrolyten angeordnete Elektrode aus dotiertem Platin, wobei der Festkörperelektrolyt als Sauerstoffionenleiter Sauerstoffionen durch sich hindurch als Festkörperelektrolyt transportiert, dient als Referenzelektrode, wobei diese erfindungsgemäß in der geschlossenen Kammer in dem Gassensor angeordnet ist.

Die geschlossene Kammer wird dadurch gebildet, dass gegenüberliegend zur Elektrode aus dotiertem Platin, die unterhalb des Festkörperelektrolyten angeordnet ist, eine gegenüberliegende und einen Abstand aufweisende elektrisch isolierenden Schicht, beispielsweise ein Plättchen oder ein Körper aus ZrO₂ oder Al₂O₃, angeordnet ist. Die elektrisch isolierende Schicht kann auch noch im Kammerbereich zusätzlich eine Glasspassivierung aufweisen.

Die Abdichtung der Kammer erfolgt aufgrund des Abstandes an den jeweiligen Seiten oder Seitenwänden beispielsweise mit Hilfe einer Glasversiegelung und/oder einer Platinpaste, die an den Seiten oder Seitenwänden jeweils angeordnet sind. Besonders bevorzugt weist die Kammer sowohl eine Glasversiegelung als auch zusätzlich eine Platinpaste an den jeweiligen Seiten oder Seitenwänden der Kammer auf. Eine geschlossene Kammer, die auch unter der Bezeichnung Hohlraum bekannt ist, ist beispielsweise aus der DE 10 2007 048 049 A1 bekannt.

In einer bevorzugten Ausgestaltung weist die Elektrode, die aus der Goldlegierung besteht, eine Schichtdicke von 0,1 µm bis 50 µm, vorzugsweise etwa 5 µm, auf.

In einer weiteren vorteilhaften Ausgestaltung ist der Abstand zwischen den Elektroden auf der Oberseite des Festkörperelektrolyten zwischen 100 µm und 500 µm, vorzugsweise etwa 300 µm.

Beispielsweise sind die Elektroden mittels Siebdruck oder mittels Dispensen auf dem Festkörperelektrolyten aufbringbar.

In einer weiteren vorteilhaften Ausgestaltung sind mittels des Gassensors oxidierbare Abgaskomponenten wie Wasserstoff-Verbindungen, Stickstoff-Verbindungen, insbesondere Stickstoffoxide, Ammoniak-Verbindungen, Kohlenstoff-Verbindungen, insbesondere Kohlenmonoxide und/oder Kohlenwasserstoffe, oder dergleichen messbar.

Gemäß vorhergehenden Ausführungen ist zum einen Sauerstoff in einer Konzentration ermittelbar, wobei diese mit Hilfe der Sauerstoffpumpe in Form der beiden Elektroden aus dotiertem Platin und des Festkörperelektrolyten ermittelt wird. Mit Hilfe der Mischpotentialelektrode und der oberhalb des Festkörperelektrolyten, das heißt auf dessen Oberseite, angeordneten Elektrode aus dotiertem Platin sind dagegen oxidierbare Verbindungen messbar.

Es ist somit möglich, eine einzige Verbindung eines oxidierbaren Gases mit einem Gassensor gemäß der Erfindung zu messen oder mehrere Verbindungen von mehreren oxidierbaren Gasen in einem Gasstrom mit einem Gassensor gemäß der Erfindung zu messen.

Die Mischpotentialelektrode zur Messung der Konzentrationen von Gasen, die kein reiner Sauerstoff sind, bildet sich in dem Gassensor aus der Elektrode, die die Goldlegierung aufweist.

In einer weiteren vorteilhaften Ausgestaltung weist der Gassensor zumindest einen Heizleiter auf. Mittels des Heizleiters ist es möglich, die Betriebstemperatur des Gassensors so einzustellen, dass insbesondere bei niedrigeren Temperaturen eine präzise, schnelle und einfache Messung möglich ist. Der Heizleiter erwärmt nämlich den Gassensor, wodurch der Sauerstofftransport durch den Festkörperelektrolyten ermöglicht wird.

Der Gassensor kann auf einem keramischen Substrat, vorzugsweise ZrO₂ oder Al₂O₃, angeordnet sein. Das keramische Substrat hat eine isolierende Funktion.

Es ist möglich den Gassensor als abgassensitiven Chip zu bezeichnen, wobei der Chip dann zumindest folgende Komponenten aufweist: einen Festkörperelektrolyten vorwiegend aus ZrO₂, und zumindest drei Elektroden, davon zwei Elektroden aus dotiertem Platin und eine Elektrode aus einer Goldlegierung.

Bei dem erfindungsgemäßen Verfahren zur Messung der Konzentrationen von Gasen, insbesondere Sauerstoff und zumindest einem weiteren Gas, vorzugsweise zumindest einer oxidierbaren Abgaskomponente in Form eines weiteren Gases, wird ein Gassensor gemäß einem der Ansprüche 1 bis 5 zur Messung verwendet.

Des Weiteren betrifft die Erfindung eine Gassensorvorrichtung zur Messung der Konzentrationen von Gasen, insbesondere von Sauerstoff und zumindest von einem weiteren Gas, vorzugsweise zumindest von einer oxidierbaren Abgaskomponente in Form eines weiteren Gases, wobei diese einen Gassensor gemäß einem der Ansprüche 1 bis 5 aufweist.

Erfindungsgemäß wird ein Gassensor gemäß einem der Ansprüche 1 bis 5 in einer Verbrennungsmaschine, in einem Kraftwerk, vorzugsweise in einem Wärmekraftwerk, in einer Heizung, vorzugsweise in einer Gas- oder Ölheizung, in einem Backofen, in einem Automobil, vorzugsweise in der Abgasführung des Automobils, in einem Abgasrohr oder in einem Container, vorzugweise einem Fruchtcontainer verwendet.

Die Festkörperelektrolyten aus ZrO₂ können erfindungsgemäß teil- oder vollstabilisiert sein.

Eine Elektrode aus dotiertem Platin wird auch als Pt-Elektrode (PlatinElektrode) bezeichnet. Eine Elektrode aus einer Goldlegierung oder einer dotierten Goldlegierung wird auch als Au-Elektrode (Gold-Elektrode) bezeichnet. Eine dotierte Goldlegierung liegt vor, wenn diese zusätzlich zu dem Gold- und Platin-Anteil von 85% zu 15 % gemäß der Erfindung ergänzend zumindest auch einen Anteil von einem Festkörperelektrolyten noch aufweist.

Es ist gemäß der Erfindung möglich, dass der Festkörperelektrolyt mehr als zwei Elektroden in Summe aus einer Goldlegierung und/oder aus einem dotierten Platin auf seiner Oberseite aufweist. Es ist gemäß der Erfindung auch möglich, dass der Festkörperelektrolyt mehr als eine Elektrode in Summe aus einem dotierten Platin auf seiner Unterseite aufweist. Diese können gemäß der Erfindung jeweils dann auch unterschiedliche Zusammensetzungen aufweisen.

Die Messung der Gaskonzentrationen der oxidierbaren Gase mit Hilfe des Gassensors erfolgt über eine Nichtgleichgewichtsspannung (eine sogenannte Nicht-Nernst-Spannung) an der Mischpotentialelektrode und eine Gleichgewichtsspannung an den beiden Elektroden aus dem dotierten Platin (eine sogenannte Nernst-Spannung aufgrund des Potentials zwischen den beiden Elektroden aus dem dotierten Platin), wobei diese Spannungen die Menge an oxidierbaren Gasen an dem an dem Gassensor vorbeiströmenden Gasgemisch und somit deren Gaskonzentration ermöglichen zu bestimmen. Bevorzugt wird ein oxidierendes Gas bei drei vorhandenen Elektroden bestimmt.

Dabei wird die Gleichgewichtsspannung an den beiden Elektroden aus dem dotierten Platin mit Hilfe eines (elektrischen) Widerstandes bestimmt, der an die auf der Oberseite des Festkörperelektrolyten befindlichen Pt-Elektrode angeschlossen ist. Der (elektrische) Widerstand ist bevorzugt in einer Gassensorvorrichtung angeordnet, die auch den Gassensor aufweist.

Durch kontinuierliches Ein- und Auspumpen von Sauerstoffionen im Sinne der Ausführungen aus der DE 196 51 328 B4 lässt sich aufgrund der entstehenden Gleichgewichtsspannung die Gaskonzentration von Sauerstoff in dem Gasgemisch gemäß vorhergehenden Ausführungen in dem Abgasstrom kontinuierlich über die Gleichgewichtsspannung ermitteln.

Ferner lässt sich die Gaskonzentration von beispielsweise Kohlenmonoxid mit Hilfe des oben beschriebenen Gassensors ermitteln.

Die bei der jeweiligen Messung der Gleichgewichtsspannung in der Platinelektrode, das heißt der Elektrode aus dem dotierten Platin, und der Nichtgleichgewichtsspannung an der Elektrode aus der Goldlegierung werden beispielsweise mittels einer Datenverarbeitungsanlage so umgerechnet, dass Gewichtsprozentanteile der Gase ermittelbar und darstellbar sind.

Für die Aufbauten eines Gassensors und ferner die Messverfahren eines Gassensors wird auf die Schriften, nämlich die DE 10 2007 048 049 A1 und die DE 197 57 112 A1 und die DE 196 51 328 B4, verwiesen und Bezug genommen.

In den Figuren sind bevorzugte Ausführungsformen der Erfindung näher erläutert.

Es zeigt
Figur 1 einen Ausschnitt eines Gassensors 1 zur Messung der Konzentrationen von Gasen,
Figur 2 einen Ausschnitt eines Gassensors 1 in einer weiteren Ausgestaltung,
Figur 3 ein Rasterelektronenmikroskopbild einer erfindungsgemäßen Au-Elektrode und
Figur 4 ein Rasterelektronenmikroskopbild einer erfindungsgemäßen Pt-Elektrode.

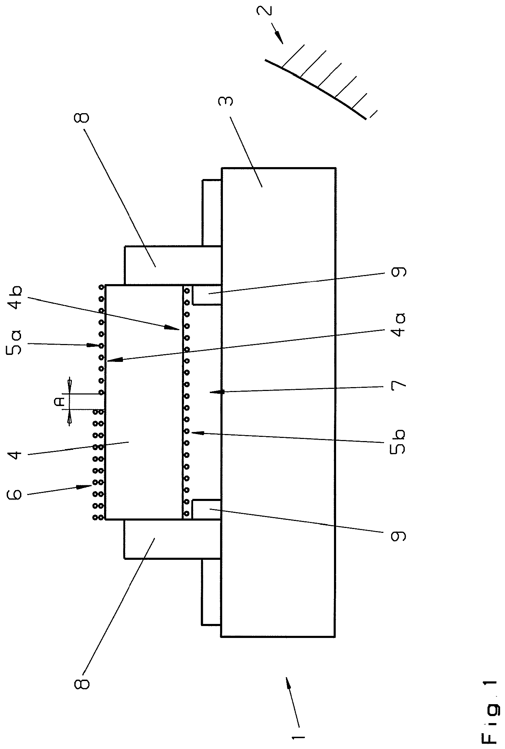

Der in Figur 1 dargestellte Gassensor 1 zur Messung der Konzentrationen von Gasen, insbesondere Sauerstoff und zumindest einem weiteren Gas, im Ausführungsbeispiel Kohlenstoffmonoxid, ist Teil einer Gassensorvorrichtung 2, die in Figur 1 angedeutet ist. Die nur angedeutete Gassensorvorrichtung 2 weist elektrische Anschlüsse, eine Steuer- oder Regeleinheit oder dergleichen auf, so dass eine Auswertung der Daten erfolgen kann, die der Gassensor 1 bei einer Messung liefert.

Es ist möglich, den Gassensor 1 zur Messung von Gaskonzentrationen beispielsweise in einem Abgasrohr einer Gas- oder Ölheizung zu verwenden, so dass beispielsweise in dem Abgasstrom die Sauerstoffkonzentration (O₂-Konzentration) und die Kohlenmonoxidkonzentration (CO-Konzentration) mittels des Gassensors 1 und somit auch der Gassensorvorrichtung 2 ermittelbar sind.

Der Gassensor 1 ist auf einem keramischen Substrat, im Ausführungsbeispiel auf einem Substrat 3 aus ZrO₂, angeordnet, das isolierend ist.

Der Gassensor 1 weist ferner einen Festkörperelektrolyten, im Ausführungsbeispiel einen Festkörperelektrolyten 4 aus zumindest 90 Gewichtsprozent ZrO₂ dotiert mit 1 bis 10 Gewichtsprozent Yttriumoxid oder 1 bis 10 Gewichtsprozent Hafniumoxid auf.

Des Weiteren weist der Gassensor 1 drei Elektroden 5a, 5b, 6 auf. Zwei Elektroden sind im Ausführungsbeispiel jeweils eine Elektrode 5a, 5b aus dotiertem Platin und die dritte Elektrode einer Elektrode 6 aus einer Goldlegierung.

Die eine Elektrode 5a aus dem dotiertem Platin ist auf der Oberseite 4a des Festkörperelektrolyten 4 angeordnet und die Elektrode 6 aus der Goldlegierung ist ebenso auf der Oberseite 4a des Festkörperelektrolyten 4 angeordnet. Die weitere Elektrode 5b aus dem dotierten Platins ist auf der Unterseite 4b des Festkörperelektrolyten 4 gemäß Figur 1 angeordnet.

Die Elektroden 5a, 5b auf dem Festkörperelektrolyten 4 bestehen jeweils im Ausführungsbeispiel aus dem gleichen dotiertem Platin, das im Ausführungsbeispiel eine Zusammensetzung von 0,5 Gewichtsprozent bis 15 Gewichtsprozent ZrO₂ und Rest Platin in Reinform aufweist. Die Elektroden 5a, 5b aus dem dotierten Platin sind bevorzugt porös ausgebildet, so dass diese Sauerstoffionen durchlassen.

Die Elektrode 6, die als Goldlegierung gemäß vorhergehenden Ausführungen ausgeführt ist, weist im Ausführungsbeispiel eine Legierungszusammensetzung von etwa 85 Gewichtsprozent Gold und etwa 15 Gewichtsprozent Platin auf. Das Verhältnis ist somit 85 % Gold zu 15 % Platin, wobei im Ausführungsbeispiel kein Festkörperelektrolyt beigemischt ist.

Alternativ ist es möglich der Elektrode 6, aufweisend eine Goldlegierung mit einem Verhältnis von 85% Gold und 15 % Platin, einen ZrO₂-Anteil beizufügen. Eine solche mit ZrO₂ dotierte Goldlegierung ist auch unter dem Begriff dotierte Goldlegierung bekannt.

Es ist möglich, dass die Goldlegierung und das dotierte Platin Verunreinigungen aufweist, die jedoch technisch zu vernachlässigen sind und bei der Herstellung entstanden sind. Ihre Anteile sind für die Konzentrationsmessungen der Gase zu vernachlässigen.

Die Elektrode 6, die aus der Goldlegierung besteht, weist im Ausführungsbeispiel eine Schichtdicke von etwa 5 µm auf.

Der Abstand A zwischen den Elektroden 5a, 6 auf der Oberseite 4a des Festkörperelektrolyten 4 ist im Ausführungsbeispiel bevorzugt etwa 300 µm.

Im Ausführungsbeispiel sind die Elektroden 5a, 5b, 6 mittels Siebdruck auf der Wand, das heißt der zugehörigen Oberseite 4a und Unterseite 4b, des Festkörperelektrolyten 4 fest aufgebracht.

Der Gassensor 1 weist ferner eine geschlossene Kammer 7 auf, wobei gemäß der Figur 1 die Unterseite 4b des Festkörperelektrolyten 4 zusammen mit der Elektrode 5b aus dem dotierten Platin einen Teil der Kammer 7 bilden. Gemäß der Figur 1 bildet somit die Elektrode 5b aus dem dotierten Platin und der Festkörperelektrolyt 4 eine Wand der Kammer 7.

Die andere Wand der Kammer 7 wird durch das Substrat 3 aus ZrO₂ gebildet. Die andere Wand weist einen Abstand in Form einer Distanz gegenüber der Unterseite 4b des Festkörperelektrolyten 4 zusammen mit der Elektrode 5b auf.

Ferner bilden sich durch den Abstand in Form der Distanz gemäß der Figur 1 Seitenwände für die Kammer 7 aus, so dass sich eine geschlossene Kammer bildet, wenn die Seitenwände verschlossen sind. Die Seitenwände sind mit Hilfe einer isolierenden Glasversiegelung 8 im Ausführungsbeispiel verschlossen. Die Seitenwände bilden dabei einen Rand beziehungsweise die Ränder der Kammer 7 im Seitenbereich. Ferner befindet sich im Bereich der Seitenwände des Gassensors 1 jeweils eine Platinpaste 9, wobei sich gemäß der Figur 1 die jeweilige Platinpaste 9 in der Kammer 7 selbst befindet.

Die Elektrode 5a ist zum Ein- und Aus-Pumpen des Sauerstoffs in die Kammer 7 vorgesehen.

In der Figur 1 ist kein Widerstand dargestellt, mittels dem mit Hilfe des Gassensors 1 die Gaskonzentration des Sauerstoffs eines zuströmenden Gasstromes gemessen werden kann.

Des Weiteren ist kein Heizleiter nach Art eines Wärmeleiters in der Figur 1 dargestellt, um beispielsweise den Gassensor 1 bei Abgasen verwenden zu können, die noch nicht eine optimale Temperatur haben, um einen optimalen lonentransport, der bei höherer Temperatur leichter und besser erfolgt, in dem Festkörperelektrolyten 4 aus ZrO₂ zu ermöglichen.

Mittels des Gassensors 1 gemäß der Figur 1 ist es möglich, eine oxidierbare Abgaskomponente, beispielsweise Kohlenstoffmonoxid, neben der Sauerstoffkonzentration beispielsweise in einem Abgasrohr zu messen. Im Ausführungsbeispiel wird nur eine einzige Abgaskomponente in Form eines oxidierbaren Gases, nämlich Kohlenmonoxid, gemessen.

Alternativ oder ergänzend ist es möglich andere Gaskonzentrationen wie die Wasserstoffkonzentration, die Stickstoffkonzentration oder dergleichen mittels des Gassensors 1 und mittels der Gassensorvorrichtung 2 zu messen.

Für eine Messung umströmt die Oberseite 4a des Festkörperelektrolyten 4 ein Gasgemisch. Die Messung der Gaskonzentrationen der oxidierbaren Gase mit Hilfe des Gassensors 1 erfolgt über eine Nichtgleichgewichtsspannung an der Mischpotentialelektrode, hier die Elektrode 6 aus der Goldlegierung, und eine Gleichgewichtsspannung an den beiden Elektroden 5a, 5b aus dem dotierten Platin, wobei diese Spannungen neben der Sauerstoffmenge die Menge an oxidierbaren Gasen, hier nur Kohlenmonoxid, an dem an dem Gassensor 1 vorbeiströmenden Gasgemisch und somit deren Gaskonzentration bestimmbar macht.

Dabei wird die Gleichgewichtsspannung an den beiden Elektroden 5a, 5b aus dem dotierten Platin mit Hilfe eines (elektrischen) Widerstandes bestimmt, der an die auf der Oberseite des Festkörperelektrolyten befindlichen Pt-Elektrode 5a angeschlossen ist. Der (elektrische) Widerstand ist bevorzugt in der Gassensorvorrichtung 2 angeordnet, die auch den Gassensor 1 aufweist. Der Widerstand ist in Figur 1 nicht dargestellt.

Die Ermittlung und Umrechnung sind aus der DE 197 57 112 A1 und ferner aus der DE 10 2007 048 049 A1 bekannt.

Der in der Figur 1 dargestellte Gassensor 1 ist mittels einer Vorrichtung zur Herstellung von Gassensoren 1 herstellbar.

Mittels eines Verfahrens zur Messung der Konzentrationen von Gasen, insbesondere Sauerstoff und zumindest einem weiteren Gas, vorzugsweise zumindest einer oxidierbaren Abgaskomponente, wird im Ausführungsbeispiel ein Gassensor 1 gemäß einem der Ansprüche 1 bis 5 zur Messung verwendet. Beispielsweise ist es möglich, den Gassensor 1 in einem Abgasrohr einer Heizung anzuordnen, wobei das Abgas des in der Heizung verbrannten Gas- oder Ölgemisches ein Gasgemisch aufweist, das mittels des Gassensors 1 messbar ist. So ist es beispielsweise möglich in dem Abgasrohr die Sauerstoffkonzentration und die Kohlenmonoxid-Konzentration des Abgases mit Hilfe des Gassensors 1 kontinuierlich zu messen.

Ferner ist ergänzend eine Anpassung der Abgaskonzentration mit Hilfe der Heizung möglich, indem die Gaskonzentration im Abgas auf optimale Werte kontinuierlich angepasst wird, so dass dadurch auch eine optimale Ausnutzung des Wirkungsgrades des der Heizung zugeführten Gas- oder Ölgemisches möglich ist, wenn die Gaszusammensetzung des Abgases kontinuierlich mit Hilfe des Gassensors 1 bekannt ist.

Es ist alternativ möglich, den vorhergehend beschriebenen Gassensor 1 in einem Kraftwerk, in einem Backofen, in einem Automobil oder in einem Container zu verwenden.

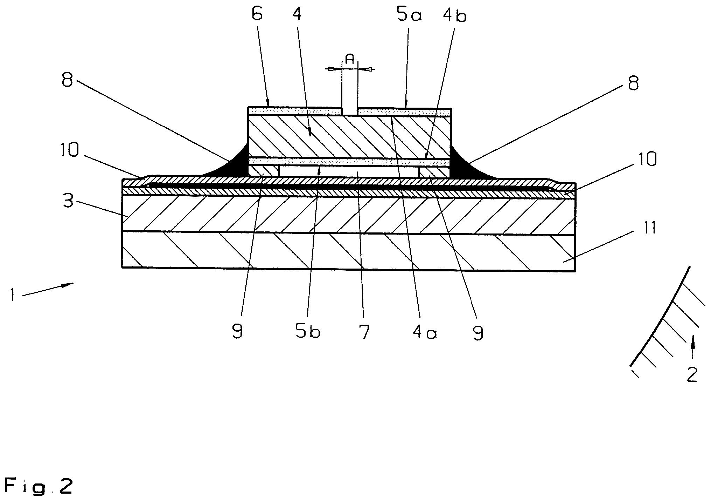

Die Verwendung des Gassensors 1 beispielsweise in einem Container ermöglicht die Lagerung der Früchte oder dergleichen in seinem solchen Container für Transporte per beispielsweise Luftweg oder Schifffahrtsweg optimal zu überwachen und einzustellen. Für den in der Figur 2 dargestellten Gassensor 1 werden ausschließlich die gegenüber der Figur 1 unterschiedlichen Merkmale beschrieben.

Gleiche Bauteile des Gassensors 1 sind in Figur 2 mit gleichen Bezugsziffern versehen und unterschiedliche Bauteile sind in Figur 2 mit neuen Bezugsziffern versehen.

Der in Figur 2 dargestellte Gassensor 1, der Teil einer Gassensorvorrichtung 2 ist, weist einen Platinheizer 11 in Form eines Heizleiters auf. Der Platinheizer 11 ist dabei auf dem Substrat 3 gemäß Figur 2 unterhalb des Substrates 3 des Gassensors 1 angeordnet. Der Platinheizer 11 ist dabei an das Substrat 3 angefügt. Ferner weist der Gassensor 1 eine zweischichtige Glaspassivierung 10 auf der Oberseite des Substrates 3 des Gassensors 1 gemäß Figur 2 auf, wobei diese Glaspassivierung 10 einen Teil der Wand der Kammer 7 gemäß Figur 2 bildet.

Mittels des Platinheizers 11 ist es möglich, die Betriebstemperatur des Gassensors 1 so einzustellen, dass insbesondere bei niedrigeren Temperaturen eine präzise, schnelle und einfache Messung der Abgaszusammensetzung möglich ist. Der Heizleiter erwärmt nämlich den Gassensor 1, wenn die Temperatur zu gering für eine optimale und schnelle Messung ist.

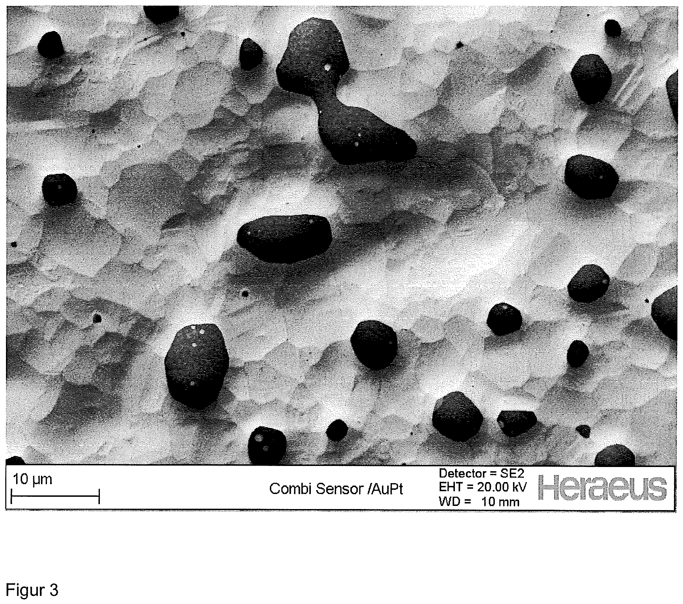

Figur 3 zeigt eine Aufnahme eines Rasterelektronenmikroskops in einer 1500-fachen Vergrößerung. Dargestellt ist die Aufnahme einer Elektrode 6, die eine erfindungsgemäße Zusammensetzung gemäß Patentanspruch 1 aufweist. Die Goldlegierung weist somit Gold und Platin im Verhältnis von 85% Gold zu 15% Platin auf. Die Porosität einer solchen Elektrode 6 ist gemäß Figur 3 sehr gering.

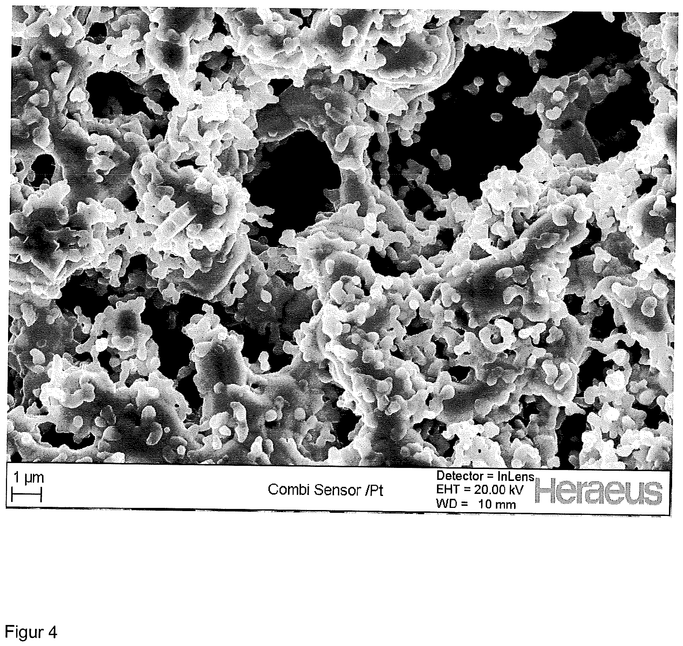

Figur 4 zeigt eine andere Aufnahme eines Rasterelektronenmikroskops in einer 3000-fachen Vergrößerung. Figur 4 zeigt dabei die Aufnahme einer Elektrode 5a, die eine erfindungsgemäße Zusammensetzung gemäß Patentanspruch 1 aufweist. Die Porosität einer solchen Elektrode 5a ist gemäß Figur 4 sehr hoch.

Die Figur 3 und die Figur 4 sind maßstabsgetreu dargestellt.

## Patentansprüche

1. Gassensor (1) zur Messung der Konzentrationen von Gasen, insbesondere von Sauerstoff und zumindest von einem weiteren Gas, vorzugsweise zumindest von einer oxidierbaren Abgaskomponente in Form eines weiteren Gases, aufweisend zumindest einen Festkörperelektrolyten (4) aus zumindest 90 Gewichtsprozent ZrO₂ und Rest zumindest aus einem weiteren Element aus der Gruppe bestehend aus Yttriumoxid und Hafniumoxid, ferner aufweisend zumindest drei Elektroden (5a, 5b, 6), von denen zwei Elektroden (5a, 5b) dotiertes Platin aufweisen und eine Elektrode (6) eine Goldlegierung aufweist, wobei zwei Elektroden (5a, 6), die eine Elektrode (5a) aus dem dotierten Platin und die eine Elektrode (6) aus der Goldlegierung, auf einer Oberseite (4a) des Festkörperelektrolyten (4) und die andere Elektrode (5b) aus dem dotierten Platin, auf einer der Oberseite (4a) gegenüberliegenden Unterseite (4b) des Festkörperelektrolyten (4) angeordnet sind, und ferner aufweisend zumindest eine geschlossene Kammer (7), wobei die Unterseite (4b) des Festkörperelektrolyten (4) einen Teil der Kammer (7) bildet, **dadurch gekennzeichnet, dass** das dotierte Platin zwischen 0,5 Gewichtsprozent bis 15 Gewichtsprozent ZrO₂ und Rest Platin aufweist, und dass die Goldlegierung etwa 85 Gewichtsprozent Gold und etwa 15 Gewichtsprozent Platin oder die Goldlegierung ausschließlich Gold und Platin im Verhältnis von 85% Gold zu 15% Platin und zusätzlich zumindest 0,5 Gewichtsprozent bis 15 Gewichtsprozent ZrO₂ aufweist.

2. Gassensor (1) gemäß Anspruch 1, **dadurch gekennzeichnet, dass** die Elektrode (6), die aus der Goldlegierung besteht, eine Schichtdicke von 0,1 µm bis 50 µm, vorzugsweise etwa 5 µm, aufweist.

3. Gassensor (1) gemäß Anspruch 1 oder Anspruch 2, **dadurch gekennzeichnet, dass** der Abstand (A) zwischen den Elektroden (5a, 6) auf der Oberseite (4a) des Festkörperelektrolyten (4) zwischen 100 µm und 500 µm, vorzugsweise etwa 300 µm, ist.

4. Gassensor (1) gemäß einem der Ansprüche 1 bis 3, **dadurch gekennzeichnet, dass** mittels des Gassensors (1) oxidierbare Abgaskomponenten wie Wasserstoff-Verbindungen, Stickstoff-Verbindungen, insbesondere Stickstoffoxide, Ammoniak-Verbindungen, Kohlenstoff-Verbindungen, insbesondere Kohlenmonoxide und/oder Kohlenwasserstoffe, oder dergleichen messbar sind.

5. Gassensor (1) gemäß einem der Ansprüche 1 bis 4, **dadurch gekennzeichnet, dass** der Gassensor (1) zumindest einen Heizleiter aufweist.

6. Verfahren zur Messung der Konzentrationen von Gasen, insbesondere Sauerstoff und zumindest einem weiteren Gas, vorzugsweise zumindest einer oxidierbaren Abgaskomponente in Form eines weiteren Gases, **dadurch gekennzeichnet, dass** ein Gassensor (1) gemäß einem der Ansprüche 1 bis 5 zur Messung verwendet wird.

7. Gassensorvorrichtung zur Messung der Konzentrationen von Gasen, insbesondere von Sauerstoff und zumindest von einem weiteren Gas, vorzugsweise zumindest von einer oxidierbaren Abgaskomponente in Form eines weiteren Gases, **dadurch gekennzeichnet, dass** die Gassensorvorrichtung einen Gassensor (1) gemäß einem der Ansprüche 1 bis 5 aufweist.

8. Verwendung eines Gassensors (1) gemäß einem der Ansprüche 1 bis 5 in einer Verbrennungsmaschine, in einem Kraftwerk, vorzugsweise einem Wärmekraftwerk, in einer Heizung, vorzugsweise in einer Gas- oder Ölheizung, in einem Backofen, in einem Automobil, vorzugsweise in der Abgasführung des Automobils, in einem Abgasrohr oder in einem Container, vorzugsweise einem Fruchtcontainer.

## Claims

1. A gas sensor (1) for measuring the concentrations of gases, more particularly of oxygen and of at least one further gas, preferably of at least one oxidisable exhaust gas component in the form of a further gas, comprising at least one solid state electrolyte (4) consisting of at least 90 per cent by weight of ZrO₂ and a rest at least consisting of one further element from the group consisting of yttrium oxide and hafnium oxide, further comprising at least three electrodes (5a, 5b, 6), two electrodes (5a, 5b) of which have doped platinum and one electrode (6) of which has a gold alloy, wherein two electrodes (5a, 6), i.e., said one electrode (5a) of doped platinum and said one electrode (6) of the gold alloy, are arranged on a top side (4a) of the solid state electrolyte (4), and said other electrode (5b) of the doped platinum is arranged on a bottom side (4b) of the solid state electrolyte (4), said bottom side (4b) being opposite to the top side (4a), and further comprising at least one closed chamber (7), wherein the bottom side (4b) of the solid state electrolyte (4) forms a part of the chamber (7), **characterised in that** the doped platinum has between 0.5 per cent by weight to 15 per cent by weight of ZrO₂, with the rest being platinum, and that the gold alloy has about 85 per cent by weight of gold and about 15 per cent by weight of platinum, or that the gold alloy exclusively has gold and platinum in a ratio of 85% gold to 15% platinum and, in addition, at least 0.5 per cent by weight to 15 per cent by weight of ZrO₂.

2. The gas sensor (1) in accordance with Claim 1, **characterised in that** the electrode (6) that consists of the gold alloy has a layer thickness of 0.1 µm to 50 µm, preferably about 5 µm.

3. The gas sensor (1) in accordance with Claim 1 or Claim 2, **characterised in that** the distance (A) between the electrodes (5a, 6) on the top side (4a) of the solid state electrolyte (4) is between 100 µm and 500 µm, preferably about 300 µm.

4. The gas sensor (1) in accordance with any one of Claims 1 to 3, **characterised in that** oxidisable exhaust components, such as hydrogen compounds, nitrogen compounds, more particularly nitrogen oxides, ammonia compounds, carbon compounds, more particularly carbon monoxides and/or hydrocarbons, or the like, can be measured by means of the gas sensor (1).

5. The gas sensor (1) in accordance with any one of Claims 1 to 4, **characterised in that** the gas sensor (1) has at least one heating element.

6. A method for measuring the concentrations of gases, more particularly of oxygen and of at least one further gas, preferably of at least one oxidisable exhaust gas component in the form of a further gas, **characterised in that** a gas sensor (1) in accordance with any one of Claims 1 to 5 is used for the measurement.

7. Gas sensor device for measuring the concentrations of gases, more particularly of oxygen and of at least one further gas, preferably of at least one oxidisable exhaust gas component in the form of a further gas, **characterised in that** the gas sensor device has a gas sensor (1) in accordance with any one of Claims 1 to 5.

8. Use of a gas sensor (1) in accordance with any one of Claims 1 to 5 in a combustion engine, in a power station, preferably a thermal power station, in a heater, preferably in a gas or oil heater, in a baking oven, in a car, preferably in the exhaust gas routeing system of the car, in an exhaust pipe, or in a container, preferably in a fruit container.

## Revendications

1. Capteur de gaz (1) pour la mesure des concentrations de gaz, notamment d'oxygène et d'au moins un autre gaz, de préférence d'au moins un composant de gaz d'échappement oxydable sous forme d'un autre gaz, présentant au moins un électrolyte solide (4) constitué d'au moins 90 pourcent en poids de ZrO₂ et le reste au moins en un autre élément parmi le groupe constitué de l'oxyde d'yttrium et l'oxyde d'hafnium, présentant en outre au moins trois électrodes (5a, 5b, 6) parmi lesquelles deux électrodes (5a, 5b) présentent du platine dopé et une électrode (6) présente un alliage d'or, dans lequel deux électrodes (5a, 6), l'électrode (5a) en le platine dopé et l'électrode (6) en l'alliage d'or, sont disposées sur un côté supérieur (4a) de l'électrolyte solide (4) et l'autre électrode (5b) en le platine dopé est disposée sur un côté inférieur (4b) de l'électrolyte solide (4) opposé au côté supérieur (4a), et présentant en outre au moins une chambre fermée (7), dans lequel le côté inférieur (4b) de l'électrolyte solide (4) forme une partie de la chambre (7), **caractérisé en ce que** le platine dopé présente entre 0,5 pourcent en poids et 15 pourcent en poids de ZrO₂ et le reste en platine, et que l'alliage d'or présente environ 85 pourcent en poids d'or et environ 15 pourcent en poids de platine ou l'alliage d'or présente exclusivement de l'or et du platine dans le rapport de 85 % d'or sur 15 % de platine, et en outre au moins 0,5 pourcent en poids à 15 pourcent en poids de ZrO₂.

2. Capteur de gaz (1) selon la revendication 1, **caractérisé en ce que** l'électrode (6) qui se compose de l'alliage d'or présente une épaisseur de couche de 0,1 µm à 50 µm, de préférence d'environ 5 µm.

3. Capteur de gaz (1) selon la revendication 1 ou la revendication 2, **caractérisé en ce que** l'écart (A) entre les électrodes (5a, 6) sur le côté supérieur (4a) de l'électrolyte solide (4) est entre 100 µm et 500 µm, de préférence d'environ 300 µm.

4. Capteur de gaz (1) selon une des revendications 1 à 3, **caractérisé en ce que** des composants de gaz d'échappement oxydables comme des composés d'hydrogène, composés d'azote, notamment oxydes d'azote, composés d'ammoniac, composés de carbone, notamment monoxydes de carbone et/ou hydroxydes de carbone, ou similaires peuvent être mesurés au moyen du capteur de gaz (1).

5. Capteur de gaz (1) selon une des revendications 1 à 4, **caractérisé en ce que** le capteur de gaz (1) présente au moins un conducteur thermique.

6. Procédé de mesure des concentrations de gaz, notamment d'oxygène et d'au moins un autre gaz, de préférence d'au moins un composant de gaz d'échappement oxydable sous forme d'un autre gaz, **caractérisé en ce qu'**un capteur de gaz (1) selon une des revendications 1 à 5 est utilisé pour la mesure.

7. Dispositif de capteur de gaz pour la mesure des concentrations de gaz, notamment d'oxygène et d'au moins un autre gaz, de préférence d'au moins un composant de gaz d'échappement oxydable sous forme d'un autre gaz, **caractérisé en ce que** le dispositif de capteur de gaz présente un capteur de gaz (1) selon une des revendications 1 à 5.

8. Utilisation d'un capteur de gaz (1) selon une des revendications 1 à 5 dans un moteur à combustion interne, dans une centrale électrique, de préférence une centrale thermique, dans un chauffage, de préférence dans un chauffage au gaz ou fioul, dans un four, dans une automobile, de préférence dans le guidage de gaz d'échappement de l'automobile, dans un tuyau de gaz d'échappement ou dans un récipient, de préférence un récipient de fruit.
